# EUROPÄISCHE PATENTANMELDUNG

(11) **EP 4 488 656 A1**
(43) Veröffentlichungstag der Anmeldung: **08.01.2025**
(21) Anmeldenummer: 24186307.5
(22) Anmeldetag: 03.07.2024
(51) Int. Cl.: G01N 21/17, G01N 21/3504, G01N 33/00, G01N 21/359, G01N 21/61

(54) **ENERGIEOPTIMIERTES MESSVERFAHREN ZUR BESTIMMUNG EINER CO2-KONZENTRATION IN EINER LUFTATMOSPHÄRE**

(30) Priorität: 06.07.2023 DE 102023117832
(71) Anmelder: Testo SE & Co. KGaA, 79822 Titisee-Neustadt (DE)
(72) Erfinder: LANDENBERGER, Benjamin, 79102 Freiburg (DE); ZUBLER, Martin, 79853 Lenzkirch (DE); KAMPFERBECK, Michael, 79199 Kirchzarten (DE); EISENKOLB, Sarah, 79822 Titisee-Neustadt (DE)
(74) Vertreter: Mertzlufft-Paufler, Cornelius

(57) **Zusammenfassung**

Zusammenfassend wird ein Messverfahren vorgeschlagen, welches eine energieoptimierte Bestimmung von Kohlendioxid-Konzentrationen in einer Luftatmosphäre mit einem mobilen und energieautarken CO₂-Messgerät (4) erlaubt. Hierzu verfügt das CO₂-Messgerät (4) neben einem als optischen Sensor ausgestalteten CO₂-Sensor (2) einen als Halbleiter-Sensor-Chip (7) ausgestalteten Umweltsensor (3), mit dem Umweltparameter einer Luftatmosphäre (6) fortlaufend überwachbar sind. Der Betrieb des CO₂-Sensors (2) zur Messung der jeweiligen CO₂-Konzentration in der Luftatmosphäre (6) wird dabei von einem Messregler (8) des CO₂-Messgeräts (4) nur dann initiiert, wenn der Umweltsensor (3) eine signifikante Änderung des gemessenen Umweltparameters in der Luftatmosphäre (6) erfasst hat. Mit diesem Ansatz lässt sich energiesparsam über lange Zeiträume und mit ausreichender Präzision die CO₂-Konzentration in einer Luftatmosphäre (6) mit dem CO₂-Messgerät (4) bestimmen und dokumentieren (vgl. Figur 3).

## Beschreibung

Die Erfindung betrifft ein energieoptimiertes Messverfahren zur Bestimmung einer Kohlendioxid (CO₂)-Konzentration in einer Luftatmosphäre. Bei diesem Verfahren wird die CO₂-Konzentration mit Hilfe eines CO₂-Sensors ermittelt. Der CO₂-Sensor kann dabei vorzugsweise in Form eines photoakustischen Sensors ausgestaltet sein. Der besagte CO₂-Sensor ist dabei in der Lage, Kohlenstoffdioxid, also CO₂, spezifisch, also insbesondere in Abgrenzung zu anderen Gasen der Luftatmosphäre, zu messen.

Die Erfindung betrifft ferner ein Sensorsystem zum Messen einer CO₂-Konzentration in Luft. Dieses Sensorsystem umfasst einen CO₂-Sensor, der dazu eingerichtet ist, spezifisch eine Konzentration von Kohlenstoffdioxid (CO₂) innerhalb einer Luftatmosphäre zu bestimmen.

Schließlich betrifft die Erfindung ein mobiles und energieautarkes CO₂-Messgerät. Dieses Messgerät kann bevorzugt als ein Handmessgerät und/oder als ein Datenlogger ausgestaltet sein. Dieses CO₂-Messgerät weist einen elektrischen Akkumulator auf, der den gewünschten energieautarken Betrieb ermöglicht.

Zur Bewertung der Raumluftqualität (Indoor Air Quality = IAQ) wird neben Temperatur, Luftfeuchtigkeit, VOC-Konzentration und Partikelanzahl auch der CO₂-Anteil in der Luft erfasst. Die CO₂-Konzentration in der Luft ist dabei ein wichtiger Parameter, um den Grad an Verbrauchtheit der Luft bzw. das Wohlfühl-Empfinden der Anwesenden zu beurteilen, und ggf. Gegenmaßnahmen, z.B. zum Lüften, zu treffen. Die herkömmlich eingesetzten optischen Messverfahren sind teuer und weisen einen hohen Energiebedarf auf, der einen praktikablen Batteriebetrieb nicht zulässt.

Beispielsweise werden zum Messen von CO₂-Konzentrationen in einer Luftatmosphäre bislang häufig nicht-dispersive Infrarotsensoren (NDIR-Sensoren) eingesetzt. Dies sind spektroskopische Gassensoren, die infrarote Wellenlängen benutzen, um eine Gasprobe zu analysieren. Die Konzentration des gesuchten Gases wird dabei typischerweise elektro-optisch über das Ausmaß einer Absorption einer spezifischen Wellenlänge im infraroten Spektrum gemessen. Das Licht einer Infrarot-Quelle durchstrahlt dazu eine Gasprobe in einer Probenkammer, anschließend ein optisches IR-Filter und trifft dann auf einen IR-Sensor, der ein Messsignal liefert. Dieses Konzept ist etabliert und verlässlich, leidet aber unter dem Nachteil, dass diese Art von Sensoren einen vergleichsweise hohen Stromverbrauch im Dauerbetrieb zeigen. Für mobile energieautarke Anwendungen sind NDIR-Sensoren daher in der Regel ungeeignet.

Von diesem Hintergrund ausgehend möchte die Erfindung einen CO₂-Sensor zur Verfügung stellen, der in einem mobilen Messgerät, vorzugsweise ausgestaltet als Handmessgerät, im Batteriebetrieb, also autark von einer elektrischen Spannungsversorgung, betrieben werden kann. Hierbei soll der CO₂-Sensor energiesparsam ausgelegt werden.

Zur Lösung dieser Aufgabe sind erfindungsgemäß bei einem Verfahren zur Bestimmung einer CO₂-Konzentration in einer Luftatmosphäre die Merkmale von Anspruch 1 vorgesehen. Insbesondere wird somit erfindungsgemäß zur Lösung der Aufgabe bei einem wie eingangs beschriebenen Messverfahren vorgeschlagen, dass der CO₂-Sensor in einem nicht-kontinuierlichen Messbetrieb betrieben wird, dass ferner fortlaufend, insbesondere in regelmäßigen Zeitabständen, Umweltereignisse mittels eines separaten Umweltsensors überwacht (also insbesondere erfasst) werden, und dass eine jeweilige Messung der CO₂-Konzentration mit dem CO₂-Sensor erst dann erfolgt, wenn der Umweltsensor ein solches Umweltereignis detektiert.

Mit anderen Worten ist gemäß der Erfindung vorgesehen, dass der Betrieb des CO₂-Sensors durch Pausen unterbrochen wird, deren Länge und/oder zeitliche Anordnung auf Basis von Messergebnissen festgelegt wird/ist, die mit dem Umweltsensor gewonnen werden. Der CO₂-Sensor und der Umweltsensor können dabei Teil eines erfindungsgemäßen Sensorsystems sein, wie es weiter unten noch genauer beschrieben wird.

Der zusätzlich zum CO₂-Sensor vorgesehene Umweltsensor kann vorzugsweise auf einem Halbleiter-Sensor-Chip basieren und/oder als ein Metalloxid-Halbleiter-Sensor ausgestaltet sein. Denn so können vergleichsweise energieeffizient und kostengünstig Umweltereignisse in ausreichender Frequenz überwacht werden.

Von Vorteil ist bei dem erfindungsgemäßen Verfahren, dass eine momentane zeitliche Frequenz an Messungen, die mit dem CO₂-Sensor durchgeführt werden, je nach Erfordernissen angepasst, also variabel ausgestaltet werden kann. Deuten die erfassten Umweltereignisse auf eine starke Messwertveränderung hin, kann die Frequenz der CO₂-Messungen erhöht werden; andernfalls kann sie hingegen abgesenkt werden, um so elektrische Energie einzusparen, die sonst für den Betrieb des CO₂-Sensors verbraucht würde. Damit kann die mögliche Betriebsdauer des CO₂-Sensors erheblich ausgedehnt werden, was bei energieautarker und mobiler Auslegung des Messsystems Vorteile für den Benutzer bietet.

Der Vorteil des erfindungsgemäßen Messverfahrens besteht somit im Reduzieren des Energiebedarfs, der zur zuverlässigen fortlaufenden Bestimmung der CO₂-Konzentration benötigt wird. Dadurch kann diese Art der CO₂-Sensorik in einem batteriebetriebenen Messgerät, insbesondere einem CO₂-Datenlogger, zum Einsatz kommen. Hierdurch kann das Messgerät über längere Zeiträumen von Stunden bis Tagen oder gar Monaten energieautark betrieben werden.

Erfindungsgemäß kann die Aufgabe auch durch weitere vorteilhafte Ausführungen gemäß den Unteransprüchen gelöst werden.

Beispielsweise kann vorgesehen sein, dass der nicht-kontinuierliche Messbetrieb dadurch gekennzeichnet ist, dass der CO₂-Sensor während des Messverfahrens im Mittel über wenigstens 30% der Zeit, vorzugsweise sogar über wenigstens 80% der Zeit, nicht betrieben wird oder zumindest in einem Energiesparmodus betrieben wird.

Der nicht-kontinuierliche Messbetrieb des CO₂-Sensors kann sich aber auch dadurch auszeichnen, dass der CO₂-Sensor während des Messverfahrens nur dann betrieben wird, wenn wenigstens ein Umweltparameter eine Änderung durchläuft. Wenn also der Umweltsensor eine solche (signifikante) Änderung des zu messenden Umweltparameters detektiert, kann diese Detektion den Messbetrieb des CO₂-Sensors triggern.

Gemäß einer vorteilhaften Ausgestaltung kann daher die Erfassung eines Umweltereignisses mit Hilfe des Umweltsensors eine Messung der CO₂-Konzentration mit dem CO₂-Sensor auslösen.

Die Erfassung des Umweltereignisses und/oder die Änderung des wenigstens einen Umweltparameters kann auf einer Vielzahl von Parametern beruhen; beispielsweise auf einer Änderung einer Gaszusammensetzung der Luftatmosphäre und/oder auf einer Änderung einer Temperatur der Luftatmosphäre und/oder auf einer Änderung einer Feuchtigkeit der Luftatmosphäre und/oder auf einer Änderung einer Konzentration von flüchtigen organischen Gasen (volatile organic compounds = VOC) in der Luftatmosphäre. Alle solche Umweltereignisse können somit von dem Sensorsystem als "Änderung eines Umweltparameters" erkannt bzw. als ein "Umweltereignis" erfasst werden. Entsprechend kann das Sensorsystem dann in Reaktion auf eine solche Änderung / Erfassung einen Betrieb des CO₂-Sensors im eigentlichen Messverfahren starten. Ansonsten wird der CO₂-Sensor hingegen gerade nicht betrieben oder zumindest in einem Schlaf-/Energiesparmodus, um so Energie zu sparen.

Die Abkürzung VOC (volatile organic compounds) bezeichnet flüchtige organische Verbindungen. Zur Stoffgruppe der VOC gehören zum Beispiel Kohlenwasserstoffe, Alkohole und organische Säuren. VOC kommen praktisch immer in der Raumluft vor. In der Regel sind die Konzentrationen einzelner VOC in Innenräumen relativ gering und gesundheitliche Beeinträchtigungen sind dann nicht zu befürchten. Einzelne VOC können unterschiedliche Wirkungen besitzen; diese Wirkungen reichen von Geruchsbelästigungen und Reizungen der Atemwege und Augen über akute Wirkungen bis hin zu Langzeitwirkungen. Dazu gehören auch Wirkungen auf das Nervensystem und Allergie auslösende oder Allergien verstärkende Eigenschaften. In Innenräumen sollte im Mittel die Gesamt-Konzentration an VOC unter 0,3 mg/m³ liegen, sofern keine Einzel-Richtwerte überschritten werden. Für zahlreiche Stoffe bzw. Stoffgruppen aus dem VOC-Spektrum sind oftmals Richtwerte vorgegeben, die dann Vorrang vor einer Summenbewertung haben.

Es sei an dieser Stelle hervorgehoben, dass das Umweltereignis nicht zwingend spezifisch bestimmt worden sein muss. Beispielsweise ist es möglich, dass das Umweltereignis unspezifisch mit dem Umweltsensor erfasst wird. In einem solchen Fall kann also unter Umständen unklar bleiben, welches genaue Ereignis, insbesondere welche Art von Änderung in der Luftatmosphäre, tatsächlich mit dem Umweltsensor erfasst worden ist. Dies ist beispielsweise der Fall, wenn der Umweltsensor auf Änderungen mehrerer Parameter anspricht (also diese als ein Umweltereignis detektieren kann).

Eine Ausgestaltung des Verfahrens sieht vor, dass Messdaten einer ersten Messung mit dem CO₂-Sensor und Messdaten einer zweiten Messung mit dem CO₂-Sensor interpoliert werden (insbesondere von dem Sensorsystem selbst), um einen momentanen Trend der gemessenen CO₂-Konzentration zu ermitteln. Alternativ oder ergänzend hierzu können auch Messdaten einer ersten Messung mit dem Umweltsensor und Messdaten einer zweiten Messung mit dem Umweltsensor interpoliert werden (insbesondere von dem Sensorsystem selbst), um einen momentanen Trend der gemessenen CO₂-Konzentration zu ermitteln. Die zeitliche Frequenz der CO₂-Messungen mit dem CO₂-Sensor kann dann auf Basis eines solchen Trends angepasst, also beispielsweise erhöht oder erniedrigt werden.

Eine bevorzugte Ausgestaltung sieht hierbei vor, dass eine erneute Messung mit dem CO₂-Sensor in Abhängigkeit des ermittelten Trends in einem vorgegebenen Zeitabstand durchgeführt wird oder aber nicht (abhängig vom Verlauf des Trends), insbesondere um so gegebenenfalls Energie einzusparen. Hierbei kann die Ermittlung des Trends in regelmäßigen Abständen durch erneute Messung mit dem CO₂-Sensor erfolgen und durch Auswertung des jeweiligen Messergebnisses kann somit der Trend (insbesondere fortlaufend) aktualisiert werden. Auch hierbei ist es bevorzugt, wenn der CO₂-Sensor zwischen den Messungen der CO₂-Konzentration nicht betrieben wird oder zumindest in einem Energiesparmodus betrieben wird.

Zur Lösung der Aufgabe wird auch ein Sensorsystem wie eingangs beschrieben vorgeschlagen. Dieses Sensorsystem kann bevorzugt dazu eingerichtet sein, ein wie zuvor erläutertes erfindungsgemäßes Messverfahren zu implementieren. Ein solches erfindungsgemäßes Sensorsystem kann sich dadurch auszeichnen, dass das Sensorsystem einen, vorzugsweise nicht-optischen, Umweltsensor umfasst. Beispielsweise kann der Umweltsensor auf einem Halbleiter-Sensor-Chip basieren. Ferner kann das Sensorsystem einen Messregler umfassen, der insbesondere in Form eines Mikrocontrollers ausgestaltet sein kann. Der Messregler ist zur Ausführung eines erfindungsgemäßen Messverfahrens eingerichtet, wie es zuvor beschrieben wurde oder gemäß einem der auf ein solches Verfahren gerichteten Ansprüche. Mit anderen Worten kann der Messregler somit den CO₂-Sensor und den Umweltsensor entsprechend ansteuern und/oder auslesen, um die einzelnen Verfahrensschritte zu implementieren.

Alternativ oder aber ergänzend zu diesen Merkmalen kann sich das erfindungsgemäße Sensorsystem aber auch dadurch auszeichnen, dass das Sensorsystem einen nicht-optischen Umweltsensor umfasst; wie bereits erwähnt kann dieser Umweltsensor vorzugsweise auf einem Halbleiter-Sensor-Chip basieren und/oder als ein Metalloxid-Halbleiter-Sensor ausgestaltet sein. Ferner kann kennzeichnend sein, dass der CO₂-Sensor hingegen als optischer Sensor ausgestaltet ist. Hierbei ist eine Ausgestaltung als ein photoakustischer Sensor und/oder als ein NDIR-Sensor bevorzugt, weil dies eine besonders genaue und effiziente (räumlich und energetisch) Erfassung der CO₂-Konzentration ermöglicht.

Schließlich kann auch vorgesehen sein, dass der (insbesondere optische) CO₂ Sensor zu einem nicht-kontinuierlichen Messbetrieb eingerichtet ist, um so Energie im Vergleich zu einem Dauerbetrieb einzusparen. In diesem nicht-kontinuierlichen Messbetrieb kann der CO₂-Sensor zum Beispiel regelmäßig und automatisiert nicht betrieben werden oder zumindest in einem Energiesparmodus betrieben werden.

Es kann ferner vorgesehen sein, dass der CO₂-Sensor, insbesondere ausschließlich, in Abhängigkeit von Messwerten des Umweltsensors zur Erfassung von Messwerten eingerichtet ist. In diesem Fall wird der CO₂-Sensor somit keinerlei Messung unabhängig von dem Umweltsensor vornehmen, sondern nur auf Basis von Messergebnissen, die der Umweltsensor liefert.

Im eigentlichen Messbetrieb kann der Umweltsensor zum Beispiel einen um wenigstens 25%, vorzugsweise um wenigstens 50%, niedrigeren elektrischen Energieverbrauch aufweisen als der CO₂-Sensor im Messbetrieb. Bei einer solchen Ausgestaltung kann also vom energiesparsameren Betrieb des Umweltsensors profitiert werden und dennoch kann eine ausreichend genaue zeitliche Auflösung der aktuellen CO₂-Konzentration erzielt werden. Unter Messbetrieb kann hier die Durchführung einer Messung mit dem jeweiligen Sensor verstanden werden, insbesondere in Abgrenzung von einem stand-by-Betrieb bei reduziertem Energieverbrauch und ohne Messung im stand-by-Betrieb; oder in Abgrenzung zu einem Versetzen des Sensors in einen Ruhezustand ganz ohne Energieverbrauch.

Bevorzugt kann der Umweltsensor des Sensorsystems wenigstens ein Metalloxid(MOX)-Halbleiter-Gassensorelement umfassen; dieses kann vorzugsweise in Form einer gassensitiven Metalloxid-Halbleiterschicht ausgestaltet sein. Der Umweltsensor kann somit insbesondere als ein Metalloxid(MOX)-Halbleiter-Sensor ausgestaltet sein.

Die Funktion eines MOX-Gassensors beruht dabei auf der Änderung der Leitfähigkeit/en seines gassensitiven MOX-Halbleiter-Gassensorelements (das insbesondere als gassensitive Dünnschicht ausgebildet sein kann) in Folge der Einwirkung von Gasen. Da mehrere Gase grundsätzlich eine solche Änderung herbeiführen können, ist die Messung notwendigerweise nicht spezifisch auf das jeweilige die Änderung verursachende Gas.

Gemäß einer besonders bevorzugten Ausgestaltung ist vorgesehen, dass der Umweltsensor wenigstens zwei, oder vorzugsweise sogar wenigstens drei, der folgenden Gase unspezifisch detektieren kann: Kohlenmonoxid (CO), Wasserstoff (H2) Stickstoffmonoxid (NO), Stickstoffdioxid (NO2), Ozon (O3), Ammoniak (NH3), Schwefeldioxid (SO2), Methan (CH4), Propan (C3H8), Alkohol (CXHYOH) (also z.B. Ethanol (C2H5OH)), oder andere volatile organic compounds (VOCs), die zuvor bereits erläutert wurden. Unspezifische Detektion heißt auch hier, dass der Umweltsensor zwar die Anwesenheit mehrerer der genannten Gase detektieren kann, aber nicht zwingend zwischen den einzelnen Gasen unterscheiden kann (zwei Gase können ein ähnliches Messergebnis des Umweltsensors auslösen).

Um die Genauigkeit der Messergebnisse zu verbessern und damit auch den energieoptimierten Betrieb des Sensorsystems zu verbessern kann insbesondere vorgesehen sein, dass der CO₂-Sensor einen integrierten Luftfeuchtesensor und einen integrierten Temperatursensor aufweist. Dadurch kann nämlich erreicht werden, dass ein von dem CO₂-Sensor geliefertes Messergebnis der CO₂-Konzentration Temperatur-kompensiert und Feuchte-kompensiert ist.

Der CO₂-Sensor des Sensorsystems kann als ein photoakustischer Sensor und/oder als ein NIR-Sensor, insbesondere als ein NDIR-Sensor, ausgestaltet sein (hierbei steht NIR für "near infrared", also den nahen infraroten Wellenlängenbereich). Mit anderen Worten kann der CO₂-Sensor somit über eine NIR-Lichtquelle verfügen, die eine Messwellenlänge im nahen infraroten Wellenlängenbereich (780 bis 3000 nm) zur Verfügung stellt.

Unter einem photoakustischen Sensor kann hier insbesondere ein Sensor verstanden werden, der auf dem Messprinzip der photoakustischen Spektroskopie (PAS) basiert. PAS ist einer der empfindlichsten Messmethoden zum Nachweis von Gasen und beruht auf dem sogenannten photoakustischen Effekt. Dabei wird einer Gasprobe in schneller Folge durch Lichtblitze Energie zugeführt. Der resultierende ständige Wechsel zwischen Erhitzung und Abkühlung in der Gasprobe (in Folge der Absorption der Lichtblitze) führt zu abwechselnder Wärmeausdehnung und -kontraktion. Diese Vibration in der Gasprobe kann unter geeigneten Umständen als Schall detektiert werden, wobei bestimmte Gase charakteristische Frequenzen an Schall erzeugen. Dadurch kann gerade CO₂ sehr spezifisch gemessen werden.

Unter einem NDIR-Sensor kann hier insbesondere ein nichtdispersiver Infrarotsensor verstanden werden. Ein solcher Sensor kann als ein spektroskopisches Messsystem verstanden werden. NDIR-Sensoren werden häufig für die Gasanalyse verwendet. Ein solcher NDIR-Sensor kann beispielsweise einen Infrarot-Strahler, eine Probenkammer, einen optischen Wellenlängenfilter und einen oder mehrere Infrarot-Detektoren umfassen.

Schließlich schlägt die Erfindung noch ein mobiles und energieautarkes CO₂-Messgerät vor, das, wie eingangs bereits erwähnt, als ein Handmessgerät und/oder als ein Datenlogger (also ein Gerät, welches Messdaten über längere Zeiträume inklusive dem jeweiligen Messzeitpunkt speichern kann) ausgestaltet werden kann. Dieses eingangs beschriebene Messgerät, welches einen elektrischen Akkumulator aufweist, der einen energieautarken Betrieb ermöglicht, zeichnet sich dadurch aus, dass das Messgerät ein erfindungsgemäßes Sensorsystem wie zuvor beschrieben oder nach einem der auf ein Sensorsystem gerichteten Ansprüche umfasst. Mit anderen Worten kann das CO₂-Messgerät, also insbesondere ein Messregler des CO₂-Messgeräts, dazu eingerichtet sein, ein erfindungsgemäßes Messverfahren gemäß einem der Verfahrensansprüche auszuführen, und zwar vorzugsweise fortlaufend.

Insbesondere dann, wenn das CO₂-Messgerät als ein Datenlogger ausgestaltet ist, kann es sich anbieten, dass das CO₂-Messgerät über einen internen Speicher verfügt und dazu eingerichtet ist, Messdaten, die mit dem CO₂-Sensor erfasst wurden, in dem Speicher abzulegen. Alternativ oder ergänzend kann das Messgerät auch derartige Messdaten über eine Datenschnittstelle ausgeben. Hierbei kann die Datenschnittstelle des Messgeräts insbesondere drahtlos ausgestaltet sein.

Die Erfindung wird nun anhand von Ausführungsbeispielen näher beschrieben, ist aber nicht auf diese Ausführungsbeispiele beschränkt. Weitere Ausbildungen der Erfindung können aus der nachfolgenden Beschreibung eines bevorzugten Ausführungsbeispiels in Verbindung mit der allgemeinen Beschreibung, den Ansprüchen sowie den Zeichnungen gewonnen werden.

Bei der folgenden Beschreibung verschiedener bevorzugter Ausführungsformen der Erfindung erhalten in ihrer Funktion übereinstimmende Elemente auch bei abweichender Gestaltung oder Formgebung übereinstimmende Bezugszahlen.

Es zeigt:
- Fig. 1: eine schematische Illustration des erfindungsgemäßen energiesparenden Ansatzes zum Bestimmen einer CO₂-Konzentration,
- Fig. 2: eine zeitliche Detailansicht eines typischen Messverlaufs bei Anwendung eines erfindungsgemäßen Messverfahrens,
- Fig. 3: eine zweite zeitliche Detailansicht eines typischen Messverlaufs bei Anwendung eines erfindungsgemäßen Messverfahrens,
- Fig. 4: eine dritte zeitliche Detailansicht eines typischen Messverlaufs bei Anwendung eines erfindungsgemäßen Messverfahrens,
- Fig. 5: eine schematische Illustration eines erfindungsgemäßen Messgeräts.

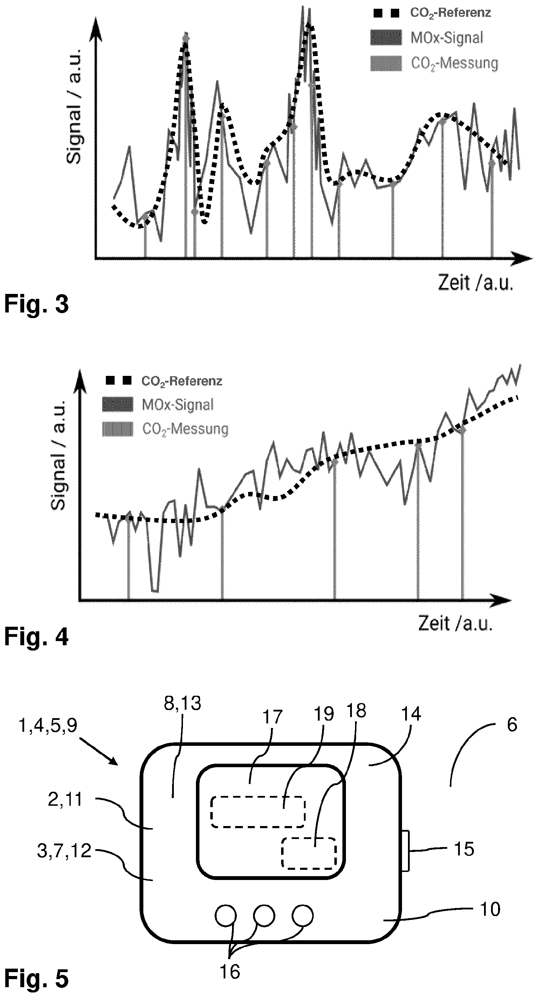

Die Figur 5 illustriert schematisch ein erfindungsgemäß ausgestaltetes mobiles und energieautarkes CO₂-Messgerät 4 in Form eines Handmessgeräts 5. Das CO₂-Messgerät 4 verfügt über einen elektrischen Akkumulator 10, der elektrische Energie zum energieautarken Betrieb des Messgeräts 4 zur Verfügung stellt. Daher kann das Messgerät 4 mobil, also unabhängig von einer stationären Spannungsversorgung, als ein Datenlogger 9 verwendet werden, der Messwerte einer CO₂-Konzentration, die mit einem CO₂-Sensor 2 des Messgeräts 4 bestimmt werden, in einem internen Speicher 14 des Messgeräts 4 ablegt. Nach erfolgter Messung können die Messwerte über eine Datenschnittstelle 15 aus dem internen Speicher 14 abgerufen werden, inklusive dem jeweiligen Messzeitpunkt.

Das Messgerät 4 umfasst nicht nur einen CO₂-Sensor 2 in Form eines photoakustischen Sensors 11, der in der Lage ist, Kohlenstoffdioxid, also CO₂, spezifisch in der das Messgerät 4 umgebenden Luftatmosphäre 6 in Abgrenzung zu anderen in dieser Luftatmosphäre 6 vorhandenen Gase zu messen, sondern auch einen Umweltsensor 3. Der zusätzliche Umweltsensor 3 basiert auf einem Halbleiter-Sensor-Chip 7 und ist als ein Metalloxid-Halbleiter-Sensor 12 ausgestaltet. Mit dem Umweltsensor 3 kann die Zusammensetzung der Luftatmosphäre 6 überwacht, d.h. sensorisch erfasst werden.

Das CO₂-Messgerät 4 der Figur 5 verfügt auch über einen als Mikrocontroller 13 ausgestalteten Messregler 8, der sowohl den CO₂-Sensor 2 als auch den Umweltsensor 3 ansteuert und ausliest. Dieser Messregler 8 implementiert somit im Zusammenspiel mit den beiden Sensoren 2, 3 ein erfindungsgemäßes Messverfahren, wobei der CO₂-Sensor nur nach Bedarf, in Abhängigkeit der mit dem Umweltsensor 3 erfassten Messsignale, zu unterschiedlichen Zeitpunkten und vor allem in unterschiedlichen Zeitabständen für eine jeweilige CO₂-Messung eingesetzt wird.

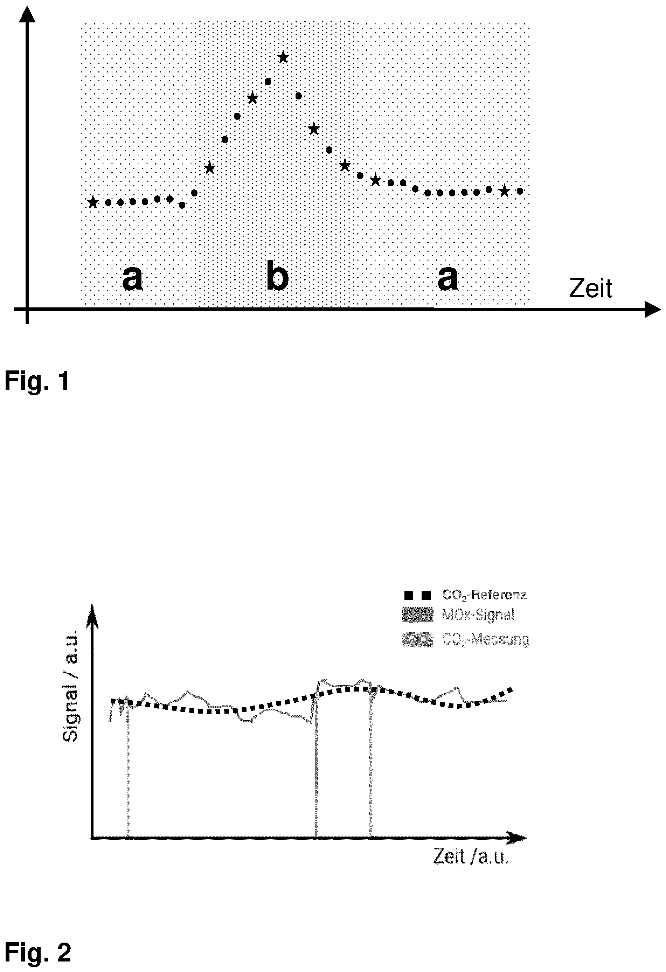

Wie anhand der schwarzen Punkte in Figur 1 illustriert ist, werden dazu mit dem Umweltsensor 3 fortlaufend (zum Beispiel in immer gleichen zeitlichen Messabständen) Messungen durchgeführt, sodass mit dem Umweltsensor 3 Umweltereignisse, also eine Änderung eines Umweltparameters in der Luftatmosphäre 6, detektiert werden können. Ein solches Umweltereignis kann zum Beispiel darin bestehen, dass sich die Gaszusammensetzung der Luftatmosphäre 6 signifikant ändert. Der Umweltsensor 3 kann aber beispielsweise auch dazu eingerichtet sein, eine Änderung einer Temperatur der Luftatmosphäre 6 oder eine Änderung einer Feuchtigkeit der Luftatmosphäre 6 oder beispielsweise eine Änderung einer Konzentration von flüchtigen organischen Gasen (VOC) in der Luftatmosphäre 6 zu detektieren.

Aufgrund der verwendeten MOX-Technologie für den Umweltsensor 3 kann dieser die fortlaufende Überwachung der Luftatmosphäre 6 äußerst energiesparsam durchführen, allerdings mit dem Nachteil, dass Umweltereignisse nur unspezifisch mit dem Umweltsensor 3 erfassbar sind. Es kann somit unter Umständen unklar bleiben, welches genaue Ereignis, zum Beispiel welche Art von Änderungen in der Luftatmosphäre 6, tatsächlich mit dem Umweltsensor 3 erfasst worden ist.

Wie gerade die Figur 1 illustriert, kann aber mit dem Umweltsensor 3 energiesparsam detektiert werden, ob es signifikante Umweltereignisse gibt oder ob die Luftatmosphäre 6 vergleichsweise statisch ist: im Feld a) der Figur 1 sind beispielsweise nur geringe Messwertveränderungen mit dem Umweltsensor 3 (schwarze Punkte) detektiert worden, während im Feld b) vergleichsweise starke Messwertveränderungen mit dem Umweltsensor 3 erfasst wurden.

Die Stern-Symbole in Figur 1 illustrieren dabei die einzelnen Messungen, die zu unterschiedlichen Zeitpunkten mit dem CO₂-Sensor 2 durchgeführt wurden. Wie zu erkennen ist, wurde die Frequenz der Messungen mit dem CO₂-Sensor 2 im Bereich b) erhöht, auf Basis der mit dem Umweltsensor 3 detektierten vergleichsweise starken Änderung der Luftatmosphäre 6. Mit anderen Worten wird hier also der CO₂-Sensor 2 in einem nicht-kontinuierlichen Messbetrieb betrieben, bei dem die einzelnen Messzeitpunkte durch unterschiedlich lange Pausen unterbrochen sind. Die Umweltereignisse werden hingegen fortlaufend in regelmäßigen Zeitabständen mit dem separaten Umweltsensor 3 erfasst.

Die jeweilige Messung der CO₂-Konzentration mit dem CO₂-Sensor 2 erfolgt dabei jeweils erst dann, wenn der Umweltsensor 3 ein Umweltereignis detektiert hat, also eine signifikante Änderung seines Messsignals. Im Ergebnis wird so der CO₂-Sensor während des Messverfahrens im Mittel über mehr als 30 % der Zeit überhaupt nicht betrieben, wodurch erheblich elektrische Energie eingespart werden kann. Denn der CO₂-Sensor 2 wird während des Messverfahrens nur dann betrieben, wenn der besagte Umweltparameter, der mit dem Umweltsensor 3 überwacht wird, eine signifikante Änderung durchläuft.

Zwischen zwei Messungen mit dem CO₂-Sensor können die Messdaten des als Halbleitergassensor ausgestalteten Umweltsensors 3 somit dazu benutzt werden, einen CO₂-Trend zu interpolieren. In diesem Fall spielt der Energieverbrauch des CO₂-Sensors nur noch eine untergeordnete Rolle, da nur noch bei Bedarf automatisiert eine Messung mit dem CO₂-Sensor durchgeführt wird. Der Vorteil dieses erfindungsgemäßen Ansatzes besteht in der Reduktion des Energiebedarfs für das gesamte Messverfahren. Es kann aber weiterhin eine zuverlässige CO₂-Bestimmung in einem batteriebetriebenen Sensorsystem 1, das beispielsweise als ein IAQ (Indoor Air Quality)-Logger ausgestaltet werden kann, gewährleistet werden.

Dieses erfindungsgemäße Konzept ist insbesondere in Figur 2 illustriert: Anhand des vom Umweltsensor 3 gelieferten MOX-Messsignals, welches mit der jeweiligen Zusammensetzung der Luftatmosphäre 6 korreliert, erkennt man, dass zu bestimmten Zeitpunkten Umweltereignisse aufgetreten sind, nämlich erkennbar anhand der signifikanten Änderung des MOX-Signals. Entsprechend hat das Sensorsystem 1 in Reaktion hierauf (genauer dessen Messregler 8, der zur Ausführung eines erfindungsgemäßen Messverfahrens eingerichtet ist) die Frequenz der CO₂-Messungen mit dem CO₂-Sensor 2 erhöht, was man an dem horizontalen Abstand der vertikalen Linien in Figur 2 erkennt, die die jeweiligen Messzeitpunkte zu den CO₂-Messungen anzeigen.

Die Figuren 3 und 4 zeigen weitere Beispiele von typischen Messsituationen, in denen das erfindungsgemäße Konzept Vorteile hinsichtlich der Energieeffizienz bietet: Die gepunktete Linie gibt dabei eine mit einem Referenzmesssystem aufgenommene CO₂-Konzentration wieder. Auch hier ist zu erkennen, dass die zeitliche Frequenz der CO₂-Messungen in Abhängigkeit des Messsignals des als MOX-Sensors ausgestalteten Umweltsensors 3 variiert wurde. Mit anderen Worten wählt das Sensorsystem 1 auf Basis der mit seinem Umweltsensor 3 erfassten Umweltereignisse die Messzeitpunkte der jeweiligen CO₂-Messung mit dem CO₂-Sensor 2 intelligent selbst aus, sodass der zeitliche Verlauf der CO₂-Konzentration mit ausreichender Genauigkeit erfasst und als Messdaten im internen Speicher 14 des CO₂-Messgeräts 4 abgelegt werden kann.

Zusammenfassend wird ein Messverfahren vorgeschlagen, welches eine energieoptimierte Bestimmung von Kohlendioxid-Konzentrationen in einer Luftatmosphäre mit einem mobilen und energieautarken CO₂-Messgerät 4 erlaubt. Hierzu verfügt das CO₂-Messgerät 4 neben einem als optischen Sensor ausgestalteten CO₂-Sensor 2 über einen als Halbleiter-Sensor-Chip 7 ausgestalteten Umweltsensor 3, mit dem Umweltparameter einer Luftatmosphäre 6 fortlaufend überwachbar sind. Der Betrieb des CO₂-Sensors 2 zur Messung der jeweiligen CO₂-Konzentration in der Luftatmosphäre 6 wird dabei von einem Messregler 8 des CO₂-Messgeräts 4 nur dann initiiert, wenn der Umweltsensor 3 eine signifikante Änderung des gemessenen Umweltparameters in der Luftatmosphäre 6 erfasst hat. Mit diesem Ansatz lässt sich energiesparsam über lange Zeiträume und mit ausreichender Genauigkeit spezifisch die CO₂-Konzentration in einer Luftatmosphäre 6 mit dem CO₂-Messgerät 4 bestimmen und dokumentieren (vgl. Figur 3).

### Bezugszeichenliste

- 1: Sensorsystem
- 2: CO₂-Sensor
- 3: Umweltsensor
- 4: mobiles CO₂-Messgerät (energieautark aufgrund seines elektrischen Akkumulators),
- 5: Handmessgerät
- 6: Luftatmosphäre, beispielsweise Raumluft
- 7: Halbleiter-Sensor-Chip
- 8: Messregler (von 1)
- 9: Datenlogger
- 10: Akkumulator (von 5)
- 11: photoakustischer Sensor und/oder NDIR-Sensor
- 12: MOX-Halbleiter-Sensor
- 13: Mikrocontroller (von 1)
- 14: Interner Speicher (von 5)
- 15: Datenschnittstelle (von 5)
- 16: Bedienelemente
- 17: Display
- 18: Temperaturanzeige
- 19: CO₂-Anzeige

## Patentansprüche

1. **Energieoptimiertes Messverfahren zur Bestimmung einer CO₂-Konzentration in einer Luftatmosphäre (6),**
- wobei die CO₂-Konzentration mit Hilfe eines CO₂-Sensors (2), vorzugsweise in Form eines photoakustischen Sensors (11), ermittelt wird,
- wobei der CO₂-Sensor (2) in der Lage ist, Kohlenstoffdioxid, also CO₂, spezifisch, insbesondere in Abgrenzung zu anderen Gasen der Luftatmosphäre (6), zu messen, **dadurch gekennzeichnet,**
- **dass** der CO₂-Sensor (2) in einem nicht-kontinuierlichen Messbetrieb betrieben wird,
- **dass** ferner fortlaufend, insbesondere in regelmäßigen Zeitabständen, Umweltereignisse mittels eines separaten Umweltsensors (3),
- vorzugsweise der auf einem Halbleiter-Sensor-Chip (7) basiert und/oder als ein Metalloxid-Halbleiter-Sensor (12) ausgestaltet ist,
überwacht werden, und
- **dass** eine jeweilige Messung der CO₂-Konzentration mit dem CO₂-Sensor (2) erst dann erfolgt, wenn der Umweltsensor (3) eine solches Umweltereignis detektiert.

2. Messverfahren nach Anspruch 1, wobei der nicht-kontinuierliche Messbetrieb **dadurch gekennzeichnet ist,**
- **dass** der CO₂-Sensor (2) während des Messverfahrens im Mittel über wenigstens 30% der Zeit, vorzugsweise über wenigstens 80% der Zeit, nicht oder in einem Energiesparmodus betrieben wird, und/oder dadurch,
- **dass** der CO₂-Sensor (2) während des Messverfahrens nur dann betrieben wird, wenn wenigstens ein Umweltparameter eine Änderung durchläuft.

3. Messverfahren nach Anspruch 1 oder 2,
- wobei die Erfassung des Umweltereignisses mit Hilfe des Umweltsensors (3) eine Messung der CO₂-Konzentration mit dem CO₂-Sensor (2) auslöst.

4. Messverfahren nach einem der vorhergehenden Ansprüche,
- wobei die Erfassung des Umweltereignisses und/oder die Änderung des wenigstens einen Umweltparameters
- auf einer Änderung einer Gaszusammensetzung der Luftatmosphäre (6) und/oder
- auf einer Änderung einer Temperatur der Luftatmosphäre (6) und/oder
- auf einer Änderung einer Feuchtigkeit der Luftatmosphäre (6) und/oder
- auf einer Änderung einer Konzentration von flüchtigen organischen Gasen (Volatile Organic Compounds = VOC) in der Luftatmosphäre (6) beruht.

5. Messverfahren nach einem der vorhergehenden Ansprüche,
- wobei das Umweltereignis unspezifisch mit dem Umweltsensor (3) erfasst wird,
- insbesondere sodass unklar bleibt, welches genaue Ereignis, insbesondere welche Art von Änderung in der Luftatmosphäre (6), tatsächlich mit dem Umweltsensor (3) erfasst worden ist.

6. Messverfahren nach einem der vorhergehenden Ansprüche,
- wobei Messdaten einer ersten Messung mit dem CO₂-Sensor (2) und Messdaten einer zweiten Messung mit dem CO₂-Sensor (2) interpoliert werden, um einen momentanen Trend der gemessenen CO₂-Konzentration zu ermitteln,
- vorzugsweise wobei eine erneute Messung mit dem CO₂-Sensor (2) in Abhängigkeit des ermittelten Trends in einem vorgegebenen Zeitabstand durchgeführt wird oder aber nicht, insbesondere um so gegebenenfalls Energie einzusparen,
- insbesondere wobei die Ermittlung des Trends in regelmäßigen Abständen durch erneute Messung mit dem CO₂-Sensor (2) und Auswertung des Messergebnisses aktualisiert wird,
- vorzugsweise wobei der CO₂-Sensor (2) zwischen den Messungen der CO₂-Konzentration nicht oder in einem Energiesparmodus betrieben wird.

7. **Sensorsystem (1) zum Messen einer CO₂-Konzentration in Luft** mit
- einem CO₂-Sensor (2) der dazu eingerichtet ist, spezifisch eine Konzentration von Kohlenstoffdioxid innerhalb einer Luftatmosphäre (6) zu bestimmen, **dadurch gekennzeichnet,**
- **dass** das Sensorsystem (1) einen, vorzugsweise nicht-optischen, Umweltsensor (3) umfasst, der auf einem Halbleiter-Sensor-Chip (7) basiert, sowie
- einen Messregler (8), insbesondere in Form eines Mikrocontrollers (14), und
- **dass** der Messregler (8) zur Ausführung eines Messverfahrens gemäß einem der vorhergehenden Ansprüche eingerichtet ist,
- vorzugsweise und hierzu den CO₂-Sensor (2) und den Umweltsensor (3) entsprechend ansteuert und/oder ausliest.

8. **Sensorsystem (1) zum Messen einer CO₂-Konzentration in Luft,** insbesondere gemäß dem vorhergehenden Anspruch, mit
- einem CO₂-Sensor (2) der dazu eingerichtet ist, spezifisch eine Konzentration von Kohlenstoffdioxid, also CO₂, innerhalb einer Luftatmosphäre (6) zu bestimmen, **dadurch gekennzeichnet,**
- **dass** das Sensorsystem (1) einen nicht-optischen Umweltsensor (3) umfasst,
- vorzugsweise der auf einem Halbleiter-Sensor-Chip (7) basiert und/oder als ein Metalloxid-Halbleiter-Sensor (12) ausgestaltet ist,
- **dass** der CO₂-Sensor (2) als optischer Sensor,
- vorzugsweise als ein photoakustischer Sensor (12) und/oder als ein NDIR-Sensor (11),
ausgestaltet ist, und
- **dass** der optische CO₂-Sensor (2) zu einem nicht-kontinuierlichen Messbetrieb eingerichtet ist,
- vorzugsweise bei welchem der CO₂-Sensor (2) regelmäßig und automatisiert nicht betrieben wird oder in einem Energiesparmodus betrieben wird.
und/oder
- zur Erfassung von Messwerten, insbesondere ausschließlich, in Abhängigkeit von Messwerten des Umweltsensors (3) eingerichtet ist.

9. Sensorsystem (1) gemäß einem der Ansprüche 7 oder 8, wobei der Umweltsensor (3) im Messbetrieb einen um wenigstens 25%, vorzugsweise um wenigstens 50%, niedrigeren elektrischen Energieverbrauch aufweist als der CO₂-Sensor (2) im Messbetrieb.

10. Sensorsystem (1) gemäß einem der Ansprüche 7 bis 9, wobei der Umweltsensor (3) wenigstens ein Metalloxid(MOX)-Halbleiter-Gassensorelement (8), vorzugsweise in Form einer gassensitiven Metalloxid-Halbleiterschicht, umfasst und/oder als ein Metalloxid(MOX)-Halbleiter-Sensor (12) ausgestaltet ist,
- insbesondere wobei der Umweltsensor (3) wenigstens zwei, vorzugsweise wenigstens drei, der folgenden Gase unspezifisch detektieren kann:
Kohlenmonoxid (CO), Wasserstoff (H2) Stickstoffmonoxid (NO), Stickstoffdioxid (NO2), Ozon (O3), Ammoniak (NH3), Schwefeldioxid (SO2), Methan (CH4), Propan (C3H8), Alkohol (CXHYOH), z.B. Ethanol (C2H5OH), oder andere Volatile Organic Compounds (VOCs).

11. Sensorsystem (1) gemäß einem der Ansprüche 7 bis 10, wobei der CO₂-Sensor (2) einen integrierten Luftfeuchtesensor und einen integrierten Temperatursensor aufweist,
- vorzugsweise sodass ein von dem CO₂-Sensor (2) geliefertes Messergebnis der CO₂-Konzentration Temperatur- und Feuchte-kompensiert ist.

12. Sensorsystem (1) gemäß einem der Ansprüche 7 bis 10, wobei der CO₂-Sensor (2) als ein photoakustischer Sensor (11) und/oder als ein NIR-Sensor (11) ausgestaltet ist,
- vorzugsweise wobei der CO₂-Sensor (2) über eine NIR-Lichtquelle verfügt.

13. **Mobiles und energieautarkes CO₂-Messgerät (4),** vorzugsweise ausgestaltet als ein Handmessgerät (5) und/oder als ein Datenlogger (9),
- wobei das CO₂-Messgerät (4) einen elektrischen Akkumulator (10) aufweist, der einen energieautarken Betrieb ermöglicht, **dadurch gekennzeichnet,**
- **dass** das CO₂-Messgerät (4) ein Sensorsystem (1) nach einem der Ansprüche 7 bis 9 umfasst und/oder
- **dass** das CO₂-Messgerät (4), insbesondere ein Messregler (8) des CO₂-Messgeräts (4), dazu eingerichtet ist, ein Messverfahren gemäß einem der Ansprüche 1 bis 6, vorzugsweise fortlaufend, auszuführen.

14. CO₂-Messgerät (4) gemäß dem vorhergehenden Anspruch,
- wobei das CO₂-Messgerät (4) als ein Datenlogger (9) ausgestaltet ist,
- wobei das CO₂-Messgerät (4) über einen internen Speicher (14) und/oder eine, insbesondere drahtlose, Datenschnittstelle (15) verfügt, und
- wobei das CO₂-Messgerät (4) dazu eingerichtet ist, Messdaten, die mit dem CO₂-Sensor (2) erfasst wurden, in dem Speicher (14) abzulegen und/oder über die Datenschnittstelle (15) auszugeben.
